# EUROPEAN PATENT APPLICATION

(11) **EP 2 415 436 A1**
(43) Date of publication of application: **08.02.2012**
(21) Application number: 10758822.0
(22) Date of filing: 31.03.2010
(51) Int. Cl.: A61F 13/15, A61F 13/472, A61F 13/49, A61F 13/53

(54) **ABSORPTIVE ARTICLE**

(30) Priority: 31.03.2009 JP 2009088494
(71) Applicant: Unicharm Corporation, Ehime 799-0111 (JP)
(72) Inventor: KOMATSU, Shinpei, Kanonji-shi Kagawa 769-1602 (JP); NODA, Yuki, Kanonji-shi Kagawa 769-1602 (JP); TANIO, Toshiyuki, Kanonji-shi Kagawa 769-1602 (JP)
(74) Representative: Eke, Philippa Dianne
(86) International application number: PCT/JP2010/055919
(87) International publication number: WO 2010/114051

(57) **Abstract**

An absorbent article includes a topsheet having a contact surface in contact with the skin of a wearer; a liquid-impermeable backsheet through which a liquid does not pass; and an absorber provided between the topsheet and the backsheet, a cooling sensation material including a refresher agent in the topsheet or between the topsheet and the backsheet. On the outside of the planar direction of the topsheet from the contact region in which an excretion unit of the wearer is in contact in the topsheet, there is a region having a greater amount of the cooling sensation material per unit area than the amount of the cooling sensation material per unit area in the contact region.

## Description

### [Technical Field]

The present invention relates to an absorbent article that includes a topsheet, a backsheet, and an absorber.

### [Background Art]

Conventionally in an absorbent article such as a disposable diaper, a cooling sensation agent that imparts a cooling sensation for the wearer is sometimes disposed in the skin contact surface that is in contact with the wearer's skin (see Patent Document 1). The cooling sensation agent is enveloped by a micro-encapsulation agent that is dissolved by water, and then placed on the skin contact surface that is in contact with the wearer's skin. When the membrane is dissolved by the wearer's urine, the components of the cooling sensation agent are released, creating a stimulus by coming into contact with the wearer's skin. The wearer can thereby be aware of time to exchange the diaper.

An absorbent article has also been disclosed in which a substance is applied to impart a refreshing sensation to the wearer, not only with the function of suggesting the "time to exchange" as described above but also with the objective of, for example, reducing the steaminess or stickiness when worn (see Patent Document 2).

However, the conventional absorbent article having the function of imparting a refreshing sensation to the wearer as described above has had the following problems. Because the refresher agent is disposed at a position that is in direct contact with the wearer's skin, some wearers feel that the stimulation is intense. Excessive stimulation gives the wearer a sense of discomfort rather than a refreshing sensation.

### [Related Art Document]

### [Patent Document]

Patent Document 1: JP-A-2008-006277 (Page 4, Fig. 1)
Patent Document 2: JP-A-2007-525245

### [Summary of Invention]

An absorbent article includes a topsheet having a contact surface in contact with the skin of a wearer; a liquid-impermeable backsheet through which a liquid does not pass; and an absorber provided between the topsheet and the backsheet, a cooling sensation material including a refresher agent in the topsheet or between the topsheet and the backsheet. On the outside in the planar direction of the topsheet from the contact region in which an excretion unit of the wearer is in contact in the topsheet, there is a region having a greater amount of the cooling sensation material per unit area than the amount of the cooling sensation material per unit area in the contact region.

### [Brief Description of Drawings]

[Fig. 1] Fig. 1 is a plan view of an absorbent article according to the embodiments.
[Fig. 2] Fig. 2 is a cross-section view taken along the F1-F1' line illustrated in Fig. 1.
[Fig. 3] Fig. 3 is a cross-section view taken along the F2-F2' line of an absorbent article 1.
[Fig. 4] Fig. 4 is a cross-section view of an absorbent article 1' at the same position as the F1-F1' line of the absorbent article 1.
[Fig. 5] Fig. 5 is a cross-section view of the absorbent article 1' at the same position as the F2-F2' line of the absorbent article 1.
[Fig. 6] Fig. 6 is a cross-section view of an absorbent article 2 at the same position as the F1-F1' line of the absorbent article 1.
[Fig. 7] Fig. 7 is a cross-section view of the absorbent article 2 at the same position as the F2-F2' line of the absorbent article 1.
[Fig. 8] Fig. 8 is a cross-section view of an absorbent article 2' at the same position as the F1-F1' line of the absorbent article 1.
[Fig. 9] Fig. 9 is a cross-section view of the absorbent article 2' at the same position as the F2-F2' line of the absorbent article 1.
[Fig. 10] Fig. 10 is a cross-section view of an absorbent article 3 at the same position as the F1-F1' line of the absorbent article 1.
[Fig. 11] Fig. 11 is a cross-section view of the absorbent article 3 at the same position as the F2-F2' line of the absorbent article 1.
[Fig. 12] Fig. 12 is a cross-section view of an absorbent article 3' at the same position as the F1-F1' line of the absorbent article 1.
[Fig. 13] Fig. 13 is a cross-section view of the absorbent article 3' at the same position as the F2-F2' line of the absorbent article 1.
[Fig. 14] Fig. 14 is a cross-section view of an absorbent article 4 at the same position as the F1-F1' line of the absorbent article 1.
[Fig. 15] Fig. 15 is a cross-section view of an absorbent article 5 at the same position as the F1-F1' line of the absorbent article 1.
[Fig. 16] Fig. 16 is a cross-section view of the absorbent article 5 at the same position as the F2-F2' line of the absorbent article 1.
[Fig. 17] Fig. 17 is a configuration diagram illustrating an arrangement device configured to arrange a cooling sensation material.
[Fig. 18] Fig. 18(a) is a diagram illustrating an application method of when conveyance is conducted by matching the longitudinal direction of a material 110 configuring the absorbent article to the conveyance direction MD.
Fig. 18(b) is a diagram illustrating the application method of when conveyance is conducted by matching the widthwise direction of the material 110 configuring the absorbent article to the conveyance direction MD.
[Fig. 19] Fig. 19 is a diagram illustrating an example of an application pattern for a cooling sensation material 100.
[Fig. 20] Fig. 20 is a diagram illustrating the step-wise method of applying the cooling sensation material 100 to the members configuring the absorbent article.
[Fig. 21] Fig. 21 is a diagram illustrating the members configuring the absorbent article in which the cooling sensation material 100 has been arranged at positions that are discontinuous in the flow direction during manufacturing.
[Fig. 22] Fig. 22 is a diagram illustrating the members configuring the absorbent article in which the cooling sensation material 100 has been arranged at positions that are discontinuous in the flow direction during manufacturing.

### [Description of Embodiments]

A description is provided for the absorbent article according to the embodiments, with reference to the drawings. In the following description of the drawings, identical or analogous parts are given identical or analogous reference numerals. It must be noted that the drawings are schematic ones, and the respective dimensional ratios and the like may differ from reality.

Accordingly the specific dimensions and the like must be determined by consulting the following description. It is a matter of course that the interrelationships of the drawings also contain parts with mutually differing dimensional ratios and relationships.

### (First Embodiment)

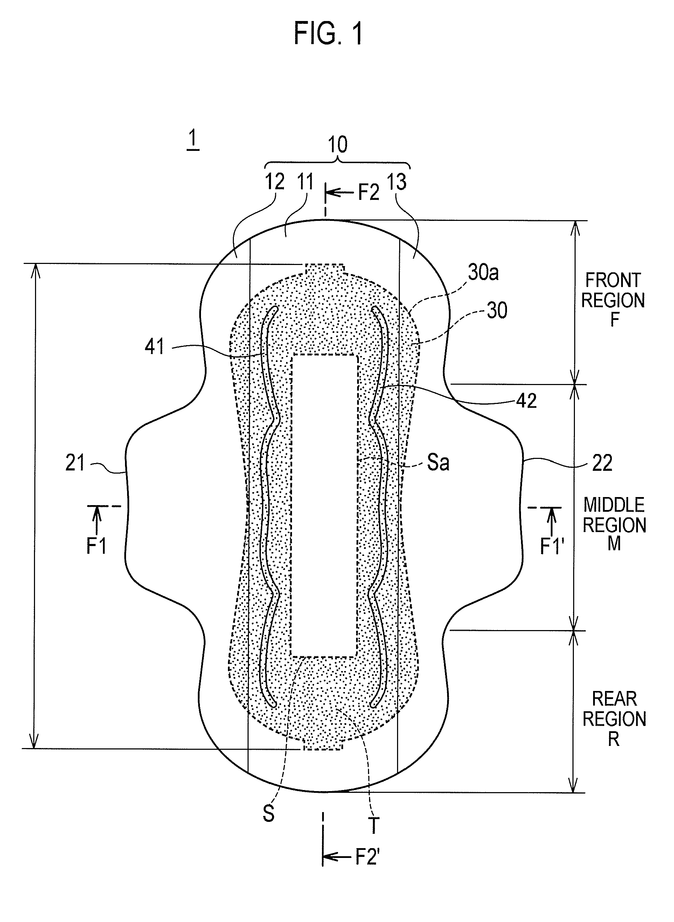

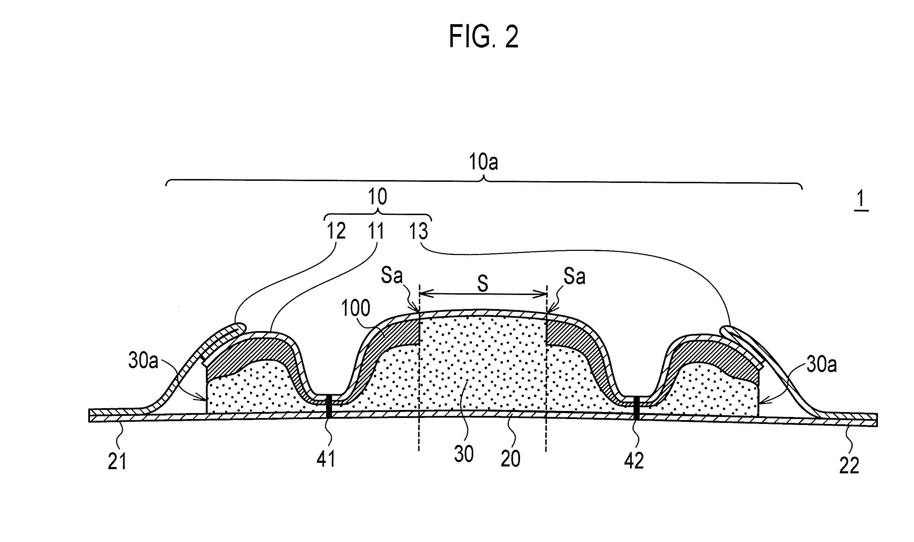

A description is provided with reference to Fig. 1 and Fig. 2 for an absorbent article according to a first embodiment. Fig. 1 is a plan view illustrating an absorbent article 1 according to the embodiment. Fig. 2 is a cross-section view taken along the F1-F1' line illustrated in Fig. 1.

The absorbent article 1 in Fig. 1 is, for example, a sanitary napkin. As illustrated in Fig. 1, the absorbent article 1 has a front region F, a middle region M, and a rear region R. The front region F is in contact with the surface of the skin on the wearer's ventral side. The middle region M is in contact with the surface of the skin around the wearer's vaginal orifice. The rear region R is in contact with the surface of the skin on the wearer's buttocks side.

The absorbent article 1 has a topsheet 10 in contact with the wearer's skin, a liquid-impermeable backsheet 20 through which liquids cannot pass, and an absorber 30. The absorbent article 1 includes a material 100 that contains a compound for giving the wearer a refreshing sensation (hereinafter, called a refresher agent) between the topsheet 10 and the backsheet 20. In this embodiment, the material that contains the refresher agent is referred to as the cooling sensation material 100.

The absorber 30 is arranged between the topsheet 10 and the backsheet 20. Therefore, the absorber 30 is illustrated by the dashed line in Fig. 1. The absorber 30 is arranged in the central portion in the longitudinal direction of the absorbent article 1.

On the outside in the planar direction of the topsheet 10 from a contact region S in contact with the wearer's excretion unit in the topsheet 10, the absorbent article 1 has a region in which the amount of the cooling sensation material 100 per unit area is greater than the amount of the cooling sensation material 100 per unit area in the contact region S.

The cooling sensation material 100 is arranged at least further inward than an edge unit 30a, and further outward than an edge unit Sa of the contact region S, in the widthwise direction of the absorber 30. The cooling sensation material 100 is arranged further inward than an edge unit 30a, and further outward than an edge unit Sa of the contact region S, in the longitudinal direction of the absorber 30. That is, between the topsheet 10 and the backsheet 20, and in the region T illustrated in Fig. 1, there is a region in which the amount of the cooling sensation material 100 per unit area is greater. The region in which the cooling sensation material 100 is arranged is a range of 30% or less further inward in the widthwise direction than the edge unit 30a of the absorber 30 relative to the length of the absorber 30 in the widthwise direction.

The topsheet 10 has a first sheet 11 that covers at least the wearer-side surface of the absorber 30, and second sheets 12 and 13 that are arranged on both sides of the widthwise direction of the first sheet 11.

The backsheet 20 has a wing 21 and a wing 22. The wing 21 and the wing 22 are formed as a pair at corresponding positions in the widthwise direction of the absorbent article 1. The wing 21 and the wing 22 extend in the widthwise direction of the absorbent article 1 in the middle region M. The width of the middle region M of the backsheet 20 is greater than the width of the front region F and the rear region R.

The first sheet 11 has substantially the same length as the length of the backsheet 20. The shape of the ends of the first sheet 11 is substantially the same as the shape of the backsheet 20. The first sheet 11 covers at least the surface of the absorber 30.

The second sheets 12 and 13 are arranged on both sides of the first sheet 11. The second sheet 12 covers the wing 21 and a part of the ends of the absorber 30. One end of the longitudinal direction of the second sheet 12 is substantially straight and overlaps with one end of the longitudinal direction of the first sheet 11. The other end of the longitudinal direction of the second sheet 12 is made to match the shape of the wing 21 and a part of the periphery of the backsheet 20.

The dimensions in the longitudinal direction of the absorbent article 1 are preferably in the range of 100 to 500 mm; specifically the range of 150 to 350 mm is even more preferable. Also, the dimensions in the widthwise direction are preferably in the range of 30 to 200 mm; specifically the range of 40 to 180 mm is even more preferable.

The second sheet 13 covers the wing 22 and a part of the end sides of the absorber 30. One end of the second sheet 13 extending in the longitudinal direction is substantially straight, and overlaps with the other end of the first sheet 11 extending in the longitudinal direction. The other end of the second sheet 13 extending in the longitudinal direction is made to match the shape of the wing 22 and a part of the periphery of the backsheet 20.

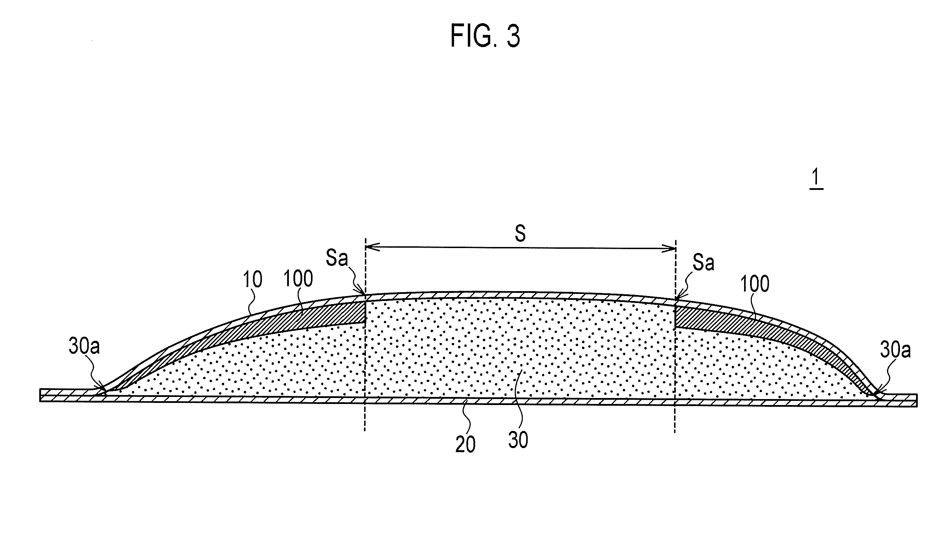

Next, a description will be provided for the positions in the thickness direction of the absorbent article 1 of the first embodiment at which the cooling sensation material 100 is arranged. Fig. 2 is a cross-section view at the F1-F1' line of the absorbent article 1. Fig. 3 is a cross-section view at the F2-F2' line of the absorbent article 1. As illustrated in Fig. 2 and Fig. 3, in the absorbent article 1, the cooling sensation material 100 is arranged in a region T illustrated in Fig. 1, between the topsheet 10 and the absorber 30.

In the absorbent article 1, the first sheet 11, the second sheets 12 and 13, the backsheet 20, and the absorber 30 are all joined together. The fringes of the first 11, the second sheets 12 and 13, and the backsheet 20 are joined together, so as to seal in the absorber 30.

Possible methods for joining the topsheet 10 and the backsheet 20 include any one of heat embossing, ultrasound, or hot-melt adhesives, or a combination of a plurality thereof. The first sheet 11 and the absorber 30 are mutually crimped by a crimp unit 41 and a crimp unit 42. The crimp unit 41 and the crimp unit 42 are formed on both sides of the width direction of the absorber 30, along the longitudinal direction of the absorber 30. In this embodiment, the crimp unit 41 and the crimp unit 42 are crimped by means of heat embossing.

An adhesive member not shown in the figures is applied in a line along the longitudinal direction of the backsheet 20 to the surface of the backsheet 20 that comes in contact with the shorts. The adhesive member is applied in a plurality of lines along the longitudinal direction of the backsheet 20. The adhesive member is also applied to the surface of the wing 21 and the wing 22 that comes in contact with the shorts. A protective sheet is bonded onto the adhesive member in order to maintain adhesiveness. The protective sheet is peeled off by the wearer at the time of use.

Next a more detailed description will be provided for the first sheets 11 and 12.

In this embodiment, the first sheet 11 is a nonwoven fabric. The raw material for the first sheet 11 is not particularly limited, provided that the same is material in a sheet-shaped structure through which liquids can pass, such as a woven fabric or a porous plastic sheet. Either natural fibers or chemical fibers can be used as the raw material of the woven or nonwoven fabric.

Examples of natural fibers include ground pulp, cotton and other types of cellulose. Examples of chemical fibers include regenerated cellulose such as rayon or fibril rayon, semi-synthetic cellulose such as acetate or triacetate, thermoplastic hydrophobic chemical fibers, or thermoplastic hydrophobic chemical fibers subjected to hydrophilic treatment.

Examples of thermoplastic hydrophobic chemical fibers include mono filaments such as polyethylene (PE), polypropylene (PP), polyethylene terephthalate (PET), fibers made by graft-polymerizing polyethylene and polypropylene, or composite fibers such as of a core-clad structure.

For the web forming method of the nonwoven fabric, it is possible to use any method of either a dry type (such as a card method, a spun bond method, a melt blown method, or an air-laid method) or a wet type. A plurality of methods may be combined from among the dry-type and wet-type methods. Further examples of methods include thermal bonding, needle punching, and chemical bonding. The method of creating the nonwoven fabric is not limited to the above methods.

A spunlace formed by means of a hydroentangling method into a sheet shape can also be used for the first sheet 11. Also, a nonwoven fabric with concavities and convexities on the upper layer side of the nonwoven fabric, or an uneven nonwoven fabric in which a non-uniform basis weight is provided in the nonwoven fabric by being hit with air at the time the web is formed can be used for the first sheet 11.
Forming concavities and convexities on the surface is able to reduce diffusion of bodily fluids going through the surface of the first sheet 11 prior to passing through the first sheet 11.

The second sheets 12 and 13 can be selected from among the same materials as the first sheet 11. However, in order to prevent the outward flow of menstrual blood from the absorbent article 1 overriding the second sheets 12 and 13, they preferably possess hydrophobicity or water-repellency Specific examples include spun bond nonwoven cloth or SMS nonwoven cloth. The second sheets 12 and 13 constitute the contact surface 10a with the skin. Therefore, in order to reduce friction irritation on the skin, it is preferable to use air-through nonwoven cloth.

Next a more detailed description will be provided for the backsheet 20.

The backsheet 20 used in this embodiment can be a film primarily composed of polyethylene, polypropylene and the like; a breathable resin film, or a sheet in which a breathable resin film is joined to a nonwoven fabric such as a spunbond or spunlace. For the backsheet 20, it is preferable to have a material possessing enough flexibility that a sense of discomfort does not occur when worn. It is preferable to use, for example, a film primarily composed of low-density polyethylene (LDPE) resin with a basis weight (weight per unit area (g)) in the range of 15 to 30 g/m².

Next, a more detailed description will be provided for the absorber 30.

The absorber 30 includes hydrophilic fibers and pulp. As examples of hydrophilic fibers, either alone or as a mixture, it is possible to use ground pulp, cellulose such as cotton, regenerated cellulose such as rayon or fibril rayon, semi-synthetic cellulose such as acetate or triacetate, a granular polymer, polymer fibers, a thermoplastic hydrophobic chemical fiber, or a thermoplastic hydrophobic chemical fiber that has been subjected to a hydrophilic treatment. Of these, considering the low costs and easy molding of the absorber, it is preferable to use ground pulp.

The absorber 30 that is used may be one in which a polymer absorber has been mixed into hydrophilic fibers. In this embodiment, the polymer absorber is a granular polymer such as sodium acrylate copolymer that is absorbent and hygroscopic. Also, the absorber 30 may include, for example, an added granular deodorizing material or granular antibacterial material such as silver, copper, zinc, silica, activated carbon, aluminosilicate compound, or zeolite. A granular cooling agent or the like may be further added, having a cooling effect by means of an endothermic reaction.

The absorber 30 may be an air-laid sheet in which a hydrophilic fiber or powder is formed into a sheet shape by an air-laid method. When an air-laid sheet is used as the absorber 30, the thickness of the sheet is preferably 0.3 to 5.0 mm. An example of air-laid sheets includes one in which a fiber or granular polymer is formed into a sheet article by a binder or the like. Note that the granular polymer in the air-laid sheet may be distributed in layers or may have a gradient in the thickness direction.

Embossing may be formed in the absorber 30 in order to prevent deformation or kinking during wearing or in order to adjust the thickness. The embossing in the absorber 30 can be formed by feeding the absorber between a patterned embossing roller and a flat roller. For the pattern of the embossing roller, a lattice, dots, waves and the like can be used, but it is preferable to use a lattice pattern, which is better able to adjust the thickness.

Next, a more detailed description will be provided for the cooling sensation material 100.

In this embodiment, the cooling sensation material 100 preferably is soluble in water and possesses a structure that encloses the refresher agent. Specifically it is preferable that the refresher agent be enclosed in polymer cells such as a clathrate compound, microcapsules, or microspheres. Examples of refresher agents include menthol, which is a derivative of cyclohexanol; camphor, and thiols. When the cooling sensation material 100 comes into contact with a liquid, the polymer cells covering the outside of the refresher agent are disintegrated, releasing the refresher agent from therein.

The cooling sensation material 100 varies according to the type of refresher agent, the type of polymer cells and the like, but is 0.1 to 50 g/m². Specifically when using 25% ratio of menthol of microspheres containing L-menthol (manufactured by Symrise AG), then 0.5 to 5 g/m² is preferable.

According to the absorbent article 1 of the embodiment as has been described above, between the topsheet 10 and the backsheet 20 and on the outside in the planar direction of the topsheet 10 from the contact region S, there is a region having a greater amount of the cooling sensation material 100 per unit area than the amount of the cooling sensation material 100 per unit area in the contact region S. For this reason, the cooling sensation material 100 does not come into direct contact with the wearer's excretion unit. Therefore, it is possible to prevent an excessive stimulus from being given to the wearer. It is also possible to more reliably suppress the occurrence of a sense of discomfort due to steaminess or stickiness.

Moreover, because of the presence of the region on the outside in the planar direction of the topsheet 10 from the contact region having a greater amount of the cooling sensation material 100 per unit area than the amount of the cooling sensation material 100 per unit area in the contact region S, the wearer senses a more intensely refreshing sensation when bodily fluids reach the outside from the contact region S. That is, increasing the intensity of the refreshing sensation signifies that the bodily fluids spread around the outer edges of the absorber 30. The cooling sensation material 100 thereby also has the role of an indicator for notifying the wearer of time to exchange the absorbent article 1.

In order to render this indicator function more effective, the cooling sensation material 100 is preferably arranged in a region that is further inward than the edge unit 30a in at least the widthwise direction of the absorber 30 and further outward than the edge unit Sa of the contact region S, and that is within 15 mm toward the central part of the absorber 30 from the edge unit 30a of the absorber 30 (including both the widthwise direction and the longitudinal direction). Within 10 mm is even more preferable.

The cooling sensation material 100 is dissolved by contact with moisture, thus releasing the refresher agent. The released refresher agent comes into contact with the cold sensitivity receptor TRPM8 (CMR1) of the skin, raising the threshold of the receptor by about 0 to 5°C. The sensible temperature of skin is thereby lowered by about 0 to 5°C without there being any change to the environmental temperature of the skin. The wearer therefore has a reduced sensible temperature in an environment in which stuffiness is sensed, and thus the wearer can receive a refreshing sensation.

### (Modification of First Embodiment)

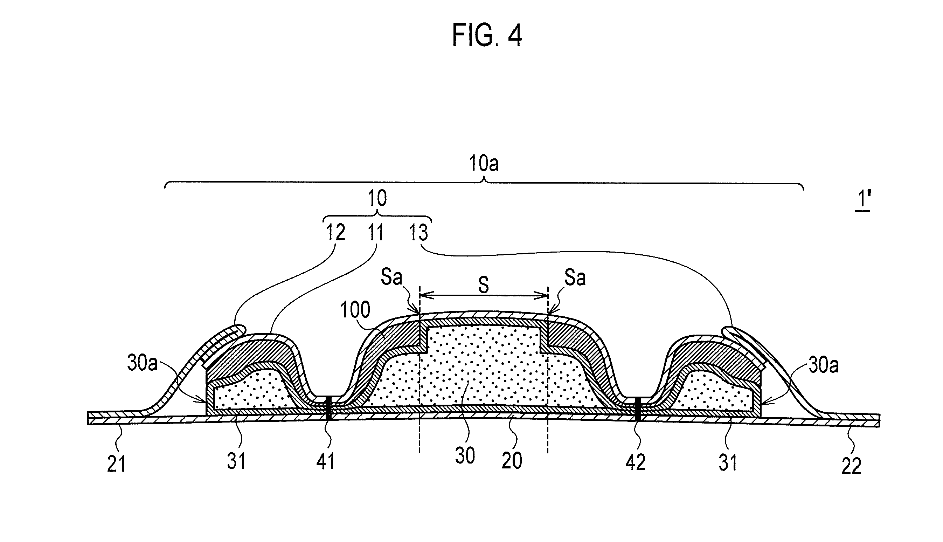

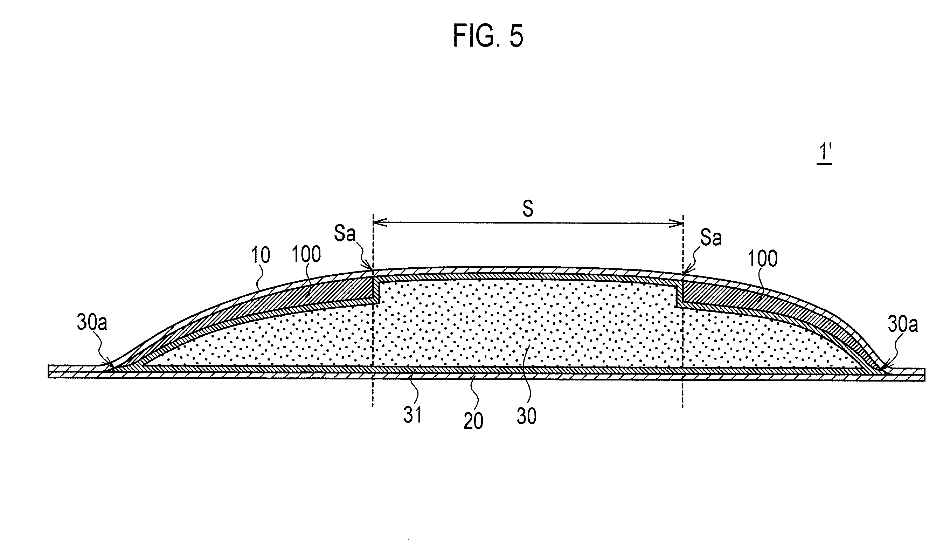

An absorbent article 1' is a modification of the first embodiment. The absorbent article 1' differs from the absorbent article 1 in the positions at which the cooling sensation material 100 is arranged in the thickness direction, but a plan view thereof has been omitted because there is no difference in the outer appearance. Fig. 4 is a cross-section view of the absorbent article 1' at the same position as the F1-F1' line of the absorbent article 1. Fig. 5 is a cross-section view of the absorbent article 1' at the same position as the F2-F2' line of the absorbent article 1. As illustrated in Fig. 4 and Fig. 5, the absorber 30 of the absorbent article 1' is one in which a hydrophilic fiber or polymer absorber or the like is covered by a covering material 31. In the absorbent article 1', between the topsheet 10 and the covering material 31, there is a region having a greater amount of the cooling sensation material 100 per unit area, at a position corresponding to the region T illustrated in Fig. 1.

In this embodiment, the cooling sensation material 100 preferably is soluble in water and possesses a structure that encloses the refresher agent. Specifically it is preferable that the refresher agent be enclosed in polymer cells such as a clathrate compound, microcapsules, or microspheres. Examples of refresher agents include menthol, which is a derivative of cyclohexanol; camphor, and thiols.

### (Second Embodiment)

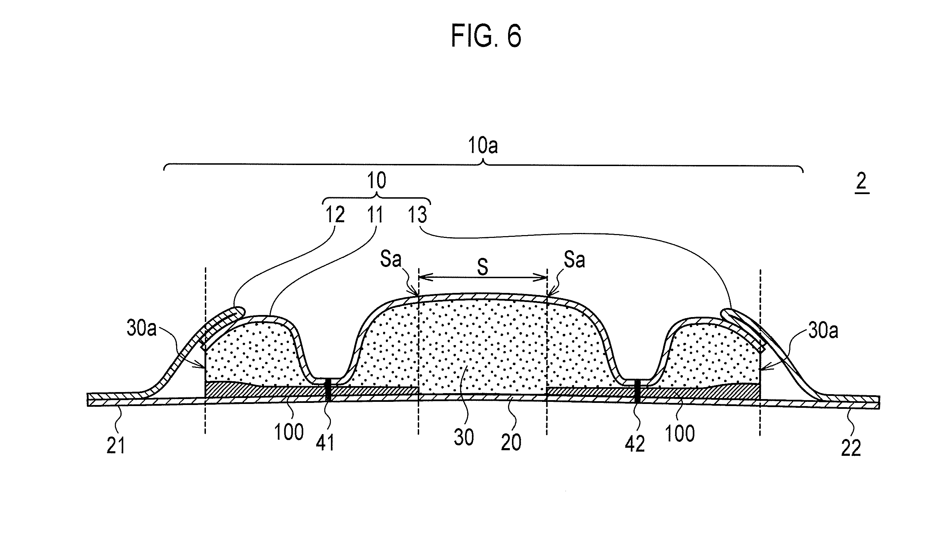

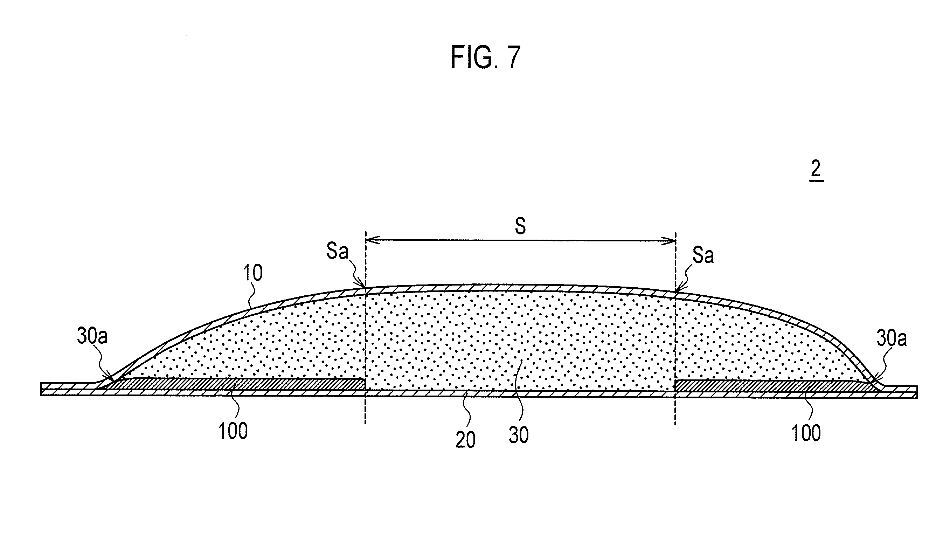

Next, a description will be provided for an absorbent article 2 illustrated as a second embodiment. The absorbent article 2 differs from the absorbent article 1 in the positions at which the cooling sensation material 100 is arranged in the thickness direction, but a plan view thereof has been omitted because there is no difference in the outer appearance. Fig. 6 is a cross-section view of the absorbent article 2 at the same position as the F1-F1' line of the absorbent article 1. Fig. 7 is a cross-section view of the absorbent article 2 at the same position as the F2-F2' line of the absorbent article 1. As illustrated in Fig. 6 and Fig. 7, in the absorbent article 2, between the absorber 30 and the backsheet 20 there is a region having a greater amount of the cooling sensation material 100 per unit area at the position corresponding to the region T illustrated in Fig. 1.

In order to render the indicator function more effective, the cooling sensation material 100 is preferably arranged in a region that is further inward than the edge unit 30a in at least the widthwise direction of the absorber 30 and further outward than the edge unit Sa of the contact region S, and that is within 15 mm toward the central part of the absorber 30 from the edge unit 30a of the absorber 30 (including both the widthwise direction and the longitudinal direction). Within 10 mm is even more preferable.

In this embodiment, the cooling sensation material 100 preferably is soluble in water and possesses a structure that encloses the refresher agent. Specifically, it is preferable that the refresher agent be enclosed in polymer cells such as a clathrate compound, microcapsules, or microspheres. Examples of refresher agents include menthol, which is a derivative of cylcohexanol; camphor, and thiols.

The cooling sensation material 100 in which the refresher agent has been enclosed in polymer cells such as a clathrate compound, microcapsules, or microspheres reacts to a liquid to release the refresher agent, and thus preferably is arranged in a layer that has a high diffusion for a liquid. In the absorbent article 2, because the cooling sensation material 100 is arranged between the absorber 30, in which the liquids are prone to accumulating, and the backsheet 20, the cooling sensation material 100 is prone to reacting with liquids and therefore there is the advantage that the refresher agent is more easily released.

Also, the cooling sensation material 100, being between the absorber 30 and the backsheet 20, does not come into direct contact with the wearer's excretion unit. Therefore, it is possible to prevent an excessive stimulus from being given to the wearer. It is further possible to more reliably suppress a sense of discomfort from stuffiness or stickiness.

### (Modification of Second Embodiment)

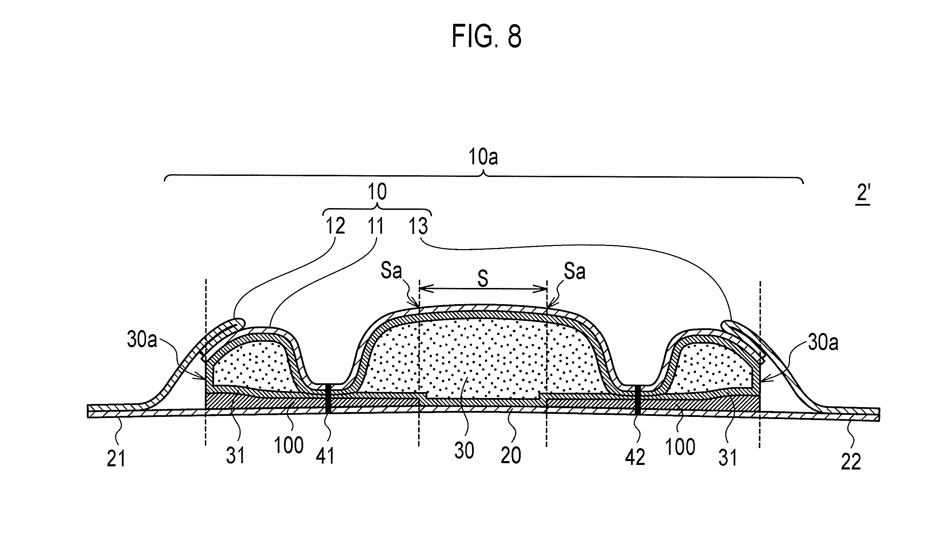

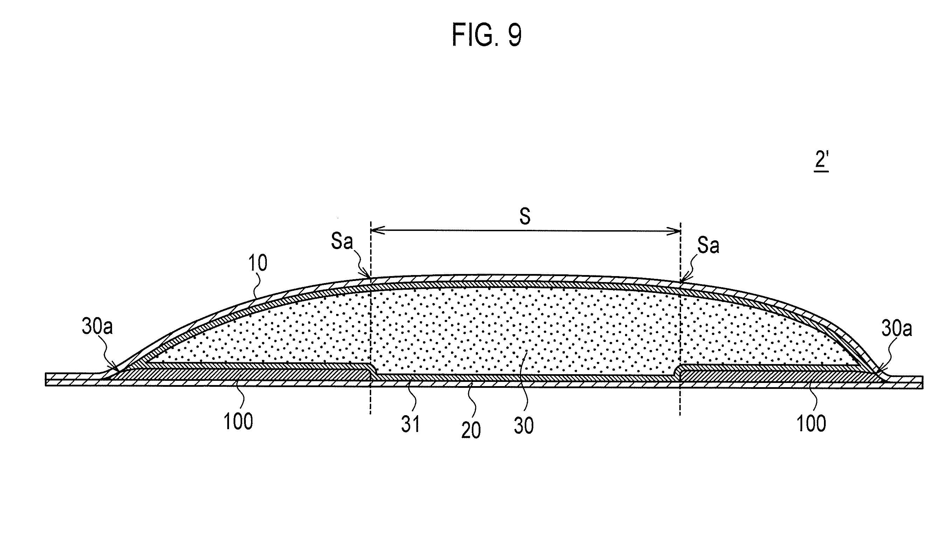

An absorbent article 2' is a modification of the second embodiment. The absorbent article 2' differs from the absorbent article 1 in the positions at which the cooling sensation material 100 is arranged in the thickness direction, but a plan view thereof has been omitted because there is no difference in the outer appearance. Fig. 8 is a cross-section view of the absorbent article 2' at the same position as the F1-F1' line of the absorbent article 1. Fig. 9 is a cross-section view of the absorbent article 2' at the same position of the F2-F2' line of the absorbent article 1. As illustrated in Fig. 8 and Fig. 9, the absorber 30 of the absorbent article 2' is one in which a hydrophilic fiber or polymer absorber or the like has been covered with a covering material 31. In the absorbent article 2', between the covering material 31 and the backsheet 20, there is a region having a greater amount of the cooling sensation material 100 per unit area at a position corresponding to the region T illustrated in Fig. 1.

### (Third Embodiment)

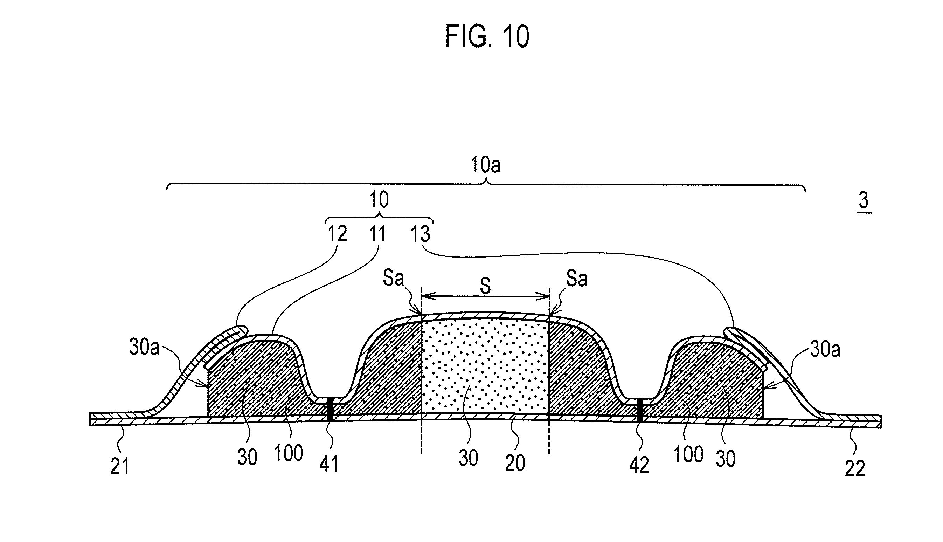

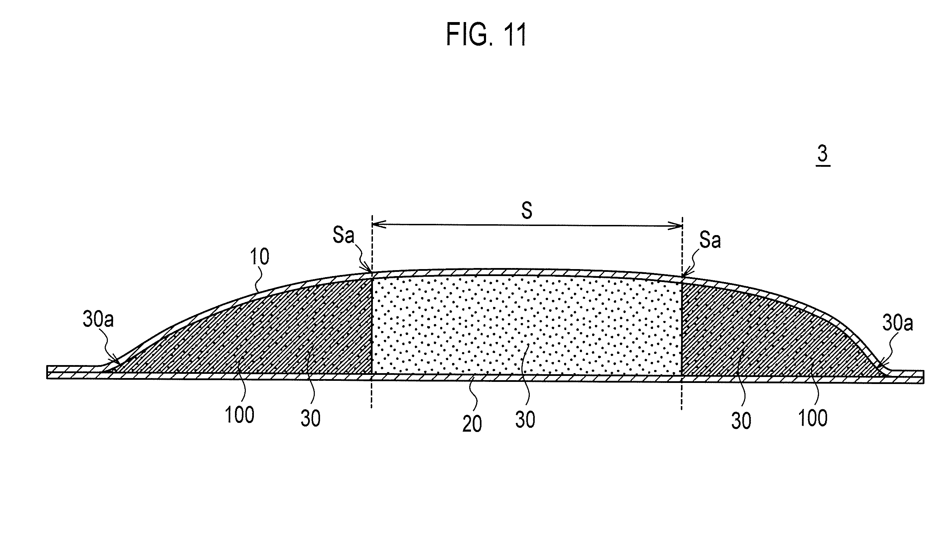

Next, a description will be provided for an absorbent article 3 illustrated as a third embodiment. The absorbent article 3 differs from the absorbent article 1 in the positions at which the cooling sensation material 100 is arranged in the thickness direction, but a plan view thereof has been omitted because there is no difference in the outer appearance. Fig. 10 is a cross-section view of the absorbent article 3 at the same position as the F1-F1' line of the absorbent article 1. Fig. 11 is a cross-section view of the absorbent article 3 at the same position as the F2-F2' line of the absorbent article 1. As illustrated in Fig. 10 and Fig. 11, in the absorbent article 3, on the inside of the absorber 30, there is a region having a greater amount of the cooling sensation material 100 per unit area at a position corresponding to the region T illustrated in Fig. 1. That is, the cooling sensation material 100 is mixed into a hydrophilic fiber or polymer absorber or the like configuring the absorber 30.

In order to render the indicator function more effective, the cooling sensation material 100 is preferably arranged in a region that is further inward than the edge unit 30a in at least the widthwise direction of the absorber 30 and further outward than the edge unit Sa of the contact region S, and that is within 15 mm toward the central part of the absorber 30 from the edge unit 30a of the absorber 30 (including both the widthwise direction and the longitudinal direction). Within 10 mm is even more preferable.

The cooling sensation material 100 in which the refresher agent has been enclosed in polymer cells such as a clathrate compound, microcapsules, or microspheres reacts to a liquid to release the refresher agent, and thus preferably is arranged in a layer that has a high diffusion for a liquid. Because the cooling sensation material 100 is arranged on the inside of the absorber 30 and reacts more readily with the liquid, the absorbent article 3 is advantageous in that the refresher agent is more readily released.

Also, the cooling sensation material 100, being arranged on the inside of the absorber 30, does not come into direct contact with the wearer's excretion unit. Therefore, it is possible to prevent an excessive stimulus from being given to the wearer. It is further possible to more reliably suppress a sense of discomfort from stuffiness or stickiness.

### (Modification of Third Embodiment)

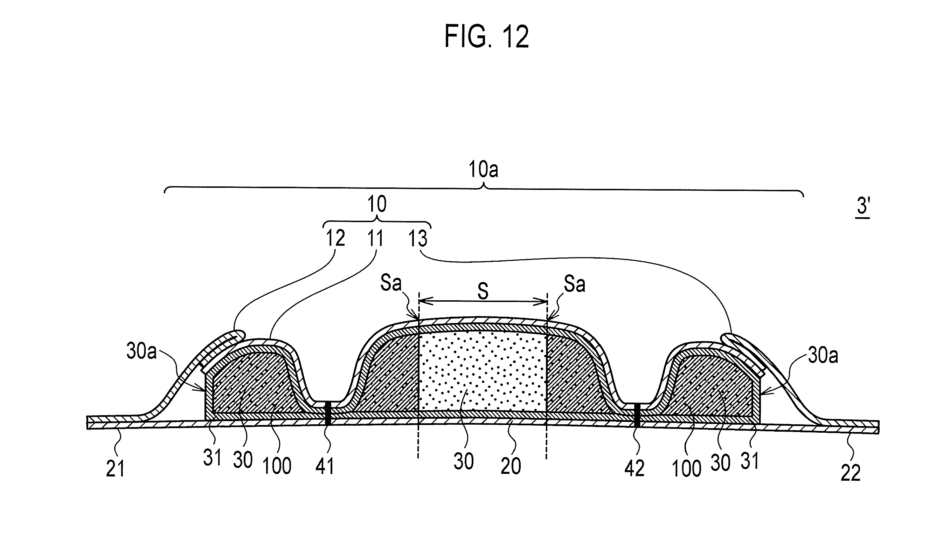

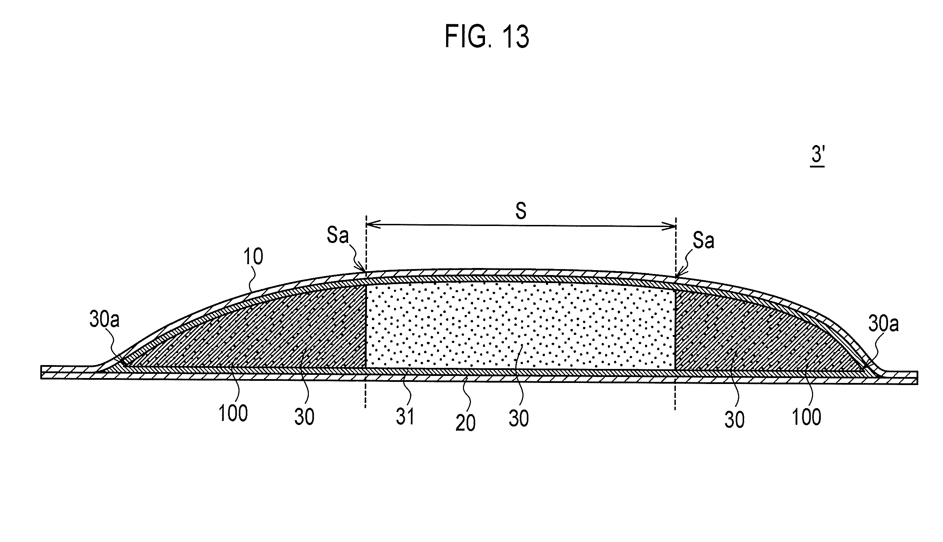

An absorbent article 3' is a modification of the third embodiment. The absorbent article 3' differs from the absorbent article 1 in the positions at which the cooling sensation material 100 is arranged in the thickness direction, but a plan view thereof has been omitted because there is no difference in the outer appearance. Fig. 12 is a cross-section view of the absorbent article 3' at the same position as the F1-F1' line of the absorbent article 1. Fig. 13 is a cross-section view of the absorbent article 3' at the same position as the F2-F2' line of the absorbent article 1. As illustrated in Fig. 12 and Fig. 13, the absorber 30 of the absorbent article 3' is one in which a hydrophilic fiber or polymer absorber or the like has been covered with a covering material 31. In the absorbent article 3', on the inside of the covering material 31, there is a region having a greater amount of the cooling sensation material 100 per unit area at a position corresponding to the region T illustrated in Fig. 1. That is, the cooling sensation material 100 is mixed into the hydrophilic fiber, polymer absorber and the like and covered by means of the covering material 31.

### (Fourth Embodiment)

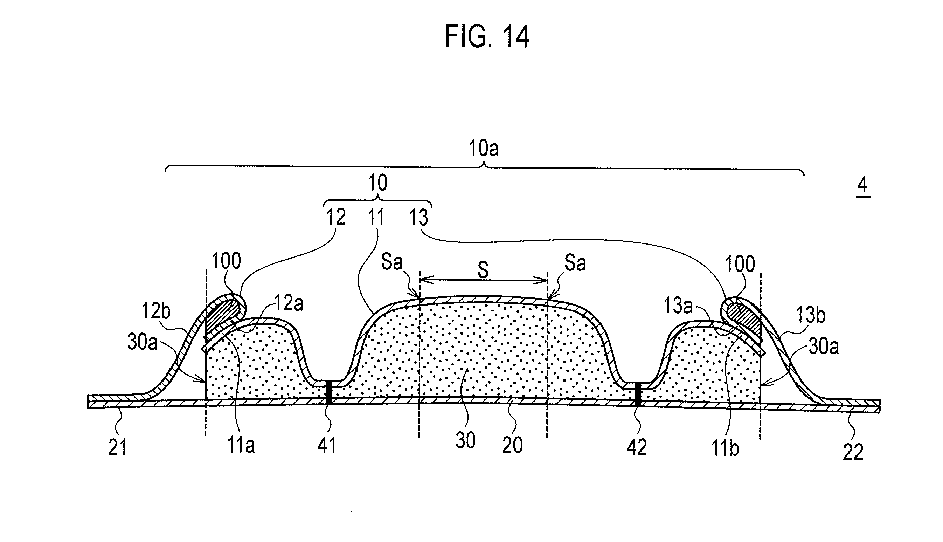

Next a description will be provided for an absorbent article 4 illustrated as a fourth embodiment. The absorbent article 4 differs from the absorbent article 1 in the positions at which the cooling sensation material 100 is arranged in the thickness direction, but a plan view thereof has been omitted because there is no difference in the outer appearance. Fig. 14 is a cross-sectional view of the absorbent article 4 at the same position as the F1-F1' line of the absorbent article 1. As illustrated in Fig. 14, in the absorbent article 4, the second sheets 12 and 13 are joined with the first sheet 11 at edge units 11a and 11b in the widthwise direction of the first sheet 11. The second sheet 12 has an edge unit 12a in the widthwise direction of the second sheet 12 and a folded-back unit 12b located outside of the edge unit 12a in the widthwise direction. A contact surface 10a that comes into contact with the wearer's skin is formed by folding back the folded-back unit 12b toward the outside of the first sheet 11 in the widthwise direction, with the second sheet 12 overlapping onto the first sheet 11 and with the edge unit 11a of the first sheet 11 in the widthwise direction and the edge unit 12a of the second sheet 12 being joined together. The second sheet 13, in a similar manner to the second sheet 12, has an edge unit 13a in the widthwise direction of the second sheet 13 and a folded-back unit 13b located outside of the edge unit 13a in the widthwise direction, and the edge unit 13a is joined to the end unit 11b of the first sheet.

The cooling sensation material 100 is arranged in a position between the edge unit 12a and the folded-back unit 12b of the second sheet 12 and corresponding to the region T illustrated in Fig. 1. When the liquid reaches this position, the absorption capacity of the absorber 30 often reaches maximum capacity. Therefore, the arranged cooling sensation material 100 functions as a so-called indicator for notifying the wearer of time to exchange. Thus, it is preferable to use a cooling sensation material 100 of such a type that the reaction with a liquid releases the refresher agent and that encloses the same in polymer cells such as of a clathrate compound, microcapsules, or microspheres. However, up until the absorption capacity reaches maximum capacity the possibility of coming into contact with a liquid is low at the position between the edge unit 12a and the folded-back unit 12b of the second sheet 12, and therefore a type of cooling sensation material 100 may be arranged in which the refresher agent is carried in a porous material. It is thereby possible to release the refresher agent by moisture without the cooling sensation material 100 coming into direct contact with a liquid.

Because the cooling sensation material 100 is located at the position where there is no direct contact with the excretion unit of the wearer, it is possible to prevent an excessive stimulus from being given to the wearer. It is further possible to more reliably suppress a sense of discomfort from stuffiness or stickiness. Moreover, it is possible to prevent moisture from seeping through to the backsides of the second sheets 12 and 13 from between the pasted sheets, compared to when the backsides of the first sheet 11 and the second sheets 12 and 13 are pasted together. Therefore, it is possible to prevent the moisture of the top surface of the first sheet 11 from coming into direct contact with the cooling sensation material 100. It is also possible to enhance the prolongableness of the refreshing action since the moisture does not come into direct contact with the cooling sensation material 100.

In the fourth embodiment, with the objective of functioning as a so-called indicator, the cooling sensation material 100 is arranged between the edge unit 12a and the folded-back unit 12b of the second sheet 12 and at a position corresponding to the region T illustrated in Fig. 1, but it is possible to combine the arrangement positions illustrated in the first through third embodiments.

### (Fifth Embodiment)

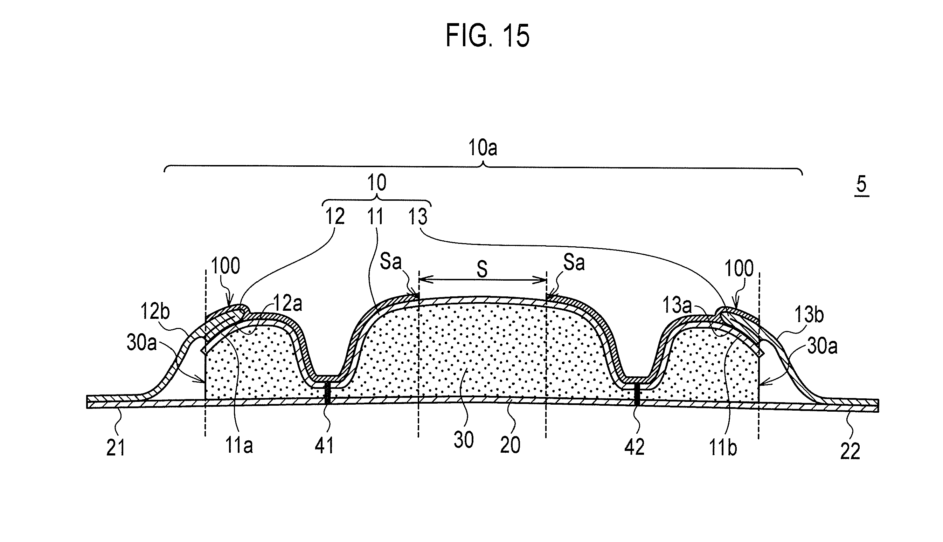

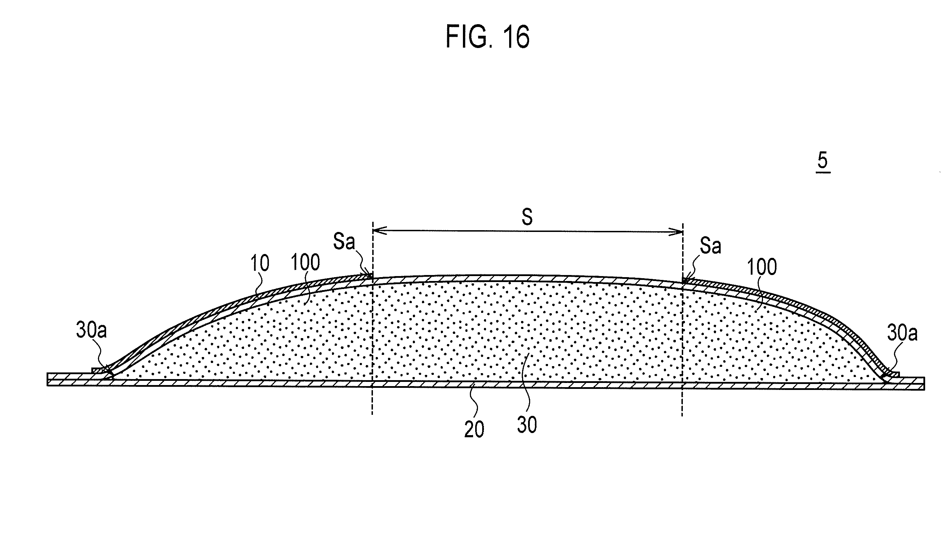

Next, a description will be provided for an absorbent article 5 illustrated as a fifth embodiment. The absorbent article 5 differs from the absorbent article 1 in the positions at which the cooling sensation material 100 is arranged in the thickness direction, but a plan view thereof has been omitted because there is no difference in the outer appearance. Fig. 15 is a cross-section view of the absorbent article 5 at the same position as the F1-F1' line of the absorbent article 1. Fig. 16 is a cross-section view of the absorbent article 5 at the same position as the F2-F2' line of the absorbent article 1. In the absorbent article 5 as illustrated in Fig. 15 and Fig. 16, in the surface of the topsheet 10, on the outside of the planar direction of the topsheet 10 from the contact region S of the topsheet 10, there is a region having a greater amount of the cooling sensation material 100 per unit area than the amount of the cooling sensation material 100 per unit area in the contact region S.

For this reason, the cooling sensation material 100 does not come into direct contact with the wearer's excretion unit. Therefore, it is possible to prevent an excessive stimulus from being given to the wearer. It is further possible to more reliably suppress a sense of discomfort from stuffiness or stickiness.

### (First Method of Arranging Cooling Sensation Agent)

The following methods are methods of arranging the cooling sensation material 100 at the positions exemplified in the above-described embodiments. The first arrangement method is a method of arranging the cooling sensation material 100 in which the refresher agent has been enclosed in polymer cells. Specifically a roller is used to bond the cooling sensation material 100 to a region to which a hot-melt adhesive has been applied.

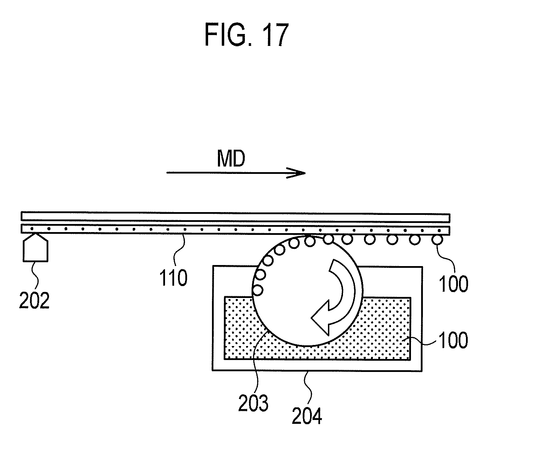

Fig. 17 is a configuration diagram illustrating the summary of the arrangement device 200 configured to arrange the cooling sensation material 100. As illustrated in Fig. 17, the arrangement device 200 has a conveyance unit 201 for conveying the material 110 in a given conveyance direction (the MD direction), and an HMA coating unit 202 for applying the surface of the material 110 with a hot-melt adhesive (hereinafter called "HMA"). Further, the arrangement device 200 has a surface treatment roller 203 configured to arrange the cooling sensation material 100 onto the surface of the material 110 applied with the HMA, and a reservoir 204 in which the cooling sensation material 100 is accumulated. Pores are formed on the surface of the surface treatment roller 203.

Rotating the surface treatment roller 203 when being in contact with the cooling sensation material 100 stored in the reservoir 204 causes the pores formed on the surface to be filled with the cooling sensation material 100. When the surface treatment roller 203 comes into contact with the surface of the material 110, the cooling sensation material 100 is transferred onto the surface of the material 110 that has been applied with HMA.

In order to partially arrange the cooling sensation material 100 to the position corresponding to the region T, the pores may be partially formed in the surface of the surface treatment roller 203. The HMA may also be applied only to the position corresponding to the region T.

The material 110 is, for example, the absorber 30. Namely by means of the arrangement device 200 of Fig. 17, the cooling sensation material 100 is alternatively arranged on the surface of the topsheet 10, at the backside of the topsheet 10 (the opposite surface of the skin contact surface in contact with the wearer's skin), or at the skin side of the absorber 30, between the absorber 30 and the backsheet 20. Because the cooling sensation material 100 is bonded to the surface of the material 110 by the HMA, therefore the amount of the cooling sensation material 100 arranged per unit area in the material 110 can be modified by modifying the application intervals of the HMA, the application area and the like.

Though depending on the types of refresher agent, on the types of the polymer cells and the like, the cooling sensation material 100 is 3 to 10 g/m² at the portion where there is a large amount to be applied, preferably 5 g/m². The portion where there is a smaller amount to be applied has 0.5 to 3 g/m², preferably 1 g/m². It is preferable that the amount of HMA applied be 5 to 100 g/m². It is also preferable to make the application width of the HMA on the order of 0.3 mm to 2 mm. In practice, the cooling sensation material 100 can be sufficiently retained when the application width is in the order of 1 mm. The region to which the HMA has been applied is preferably no greater than 2 mm, because the passage therethrough of a liquid is hindered. It is preferable that the interval between the HMA application regions be 0.3 mm or greater. When less than 0.3 mm, the liquid permeability is inferior.

Note that when a material sensitive to heat is used as the backsheet 20, it is sometimes difficult to apply the HMA to the backsheet 20. In such cases, it is preferable to arrange it onto the backsides of the second sheets 12 and 13.

The arrangement method using the arrangement device 200 as illustrated in Fig. 17 is not limited to a method of arranging the cooling sensation material 100 in which the refresher agent has been enclosed in polymer cells. The method is effective for when the cooling sensation material 100 includes granules. For example, this can be applied when arranging a certain component in which the refresher agent is carried on a porous material.

The cooling sensation material 100 that can be arranged using the first arrangement method and in which the refresher agent has been enclosed in polymer cells releases the refresher agent upon coming into contact with the liquid, and there is therefore an advantage when the cooling sensation material 100 is arranged at a place liable to come into contact with moisture such as bodily fluids. For example, it is possible to endow the same with the function of acting as an indicator. Further, when released by the cooling sensation material 100 in which the refresher agent has been carried on a porous material, the refresher agent enclosed in the micropores is replaced by the moisture (vapor) in the air. Therefore, the refreshing effect can be expressed in reaction to changes in moisture during wear, to steaminess and the like.

### (Second Method of Arranging Cooling Sensation Agent)

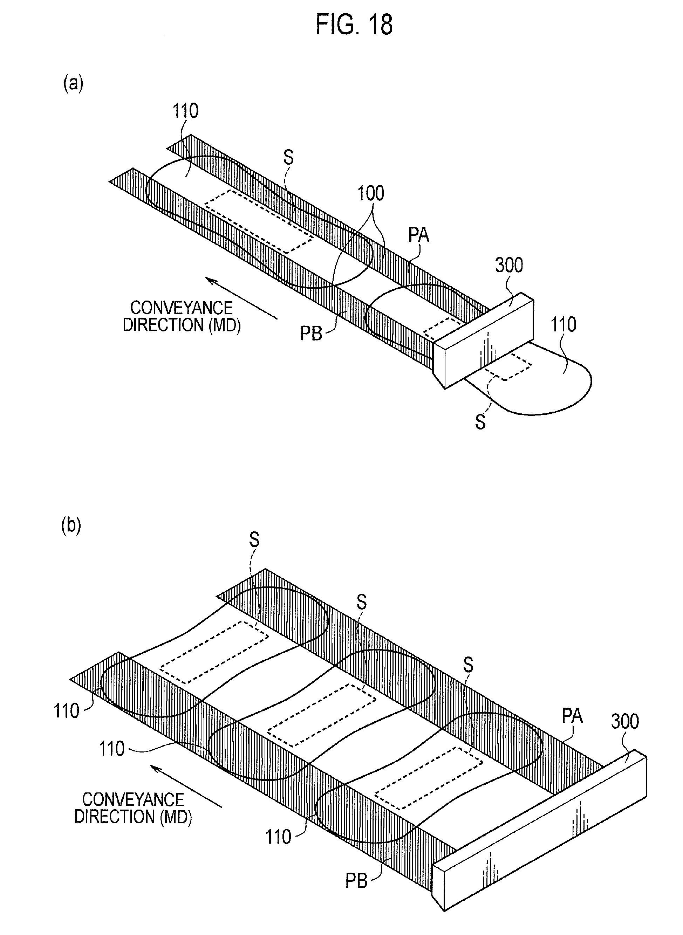

The second arrangement method is a method in which a refresher agent, such as methyl lactate and menthone glycerin acetal, soluble in a specific solvent, is applied to a given position while being a solution. When the refresher agent is a solution, a coater application can be used. Fig. 18 is a diagram illustrating the schematics of a coater application device.

Avoiding the regions on the members other than the region corresponding to the contact region S, the refresher agent is applied using the coater 300. The cooling sensation material 100 is applied to application regions PA and PB. There is the case of Fig. 18(a) in which conveyance is performed by matching the longitudinal direction of the material 110 configuring the absorbent article to the conveyance direction MD, or the case of Fig. 18(b) in which conveyance is performed by matching the widthwise direction of the material 110 configuring the absorbent article to the conveyance direction MD.

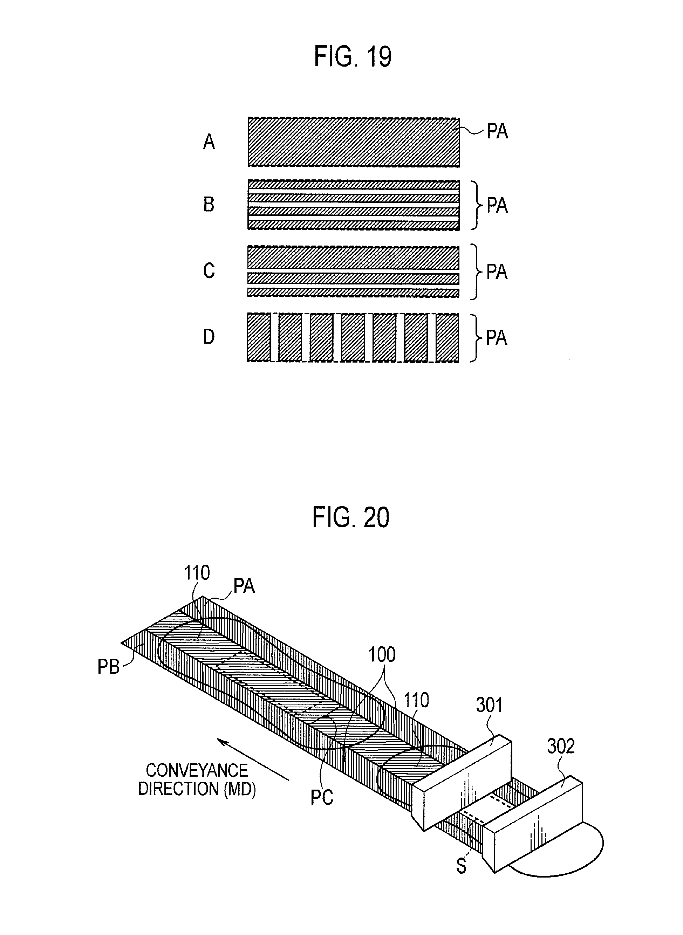

Fig. 19 illustrates an example of application patterns of the cooling sensation material 100 arranged into the application region PA. Examples include a pattern A in which the cooling sensation material 100 is applied to the entire surface of the application region PA, a pattern B in which the cooling sensation material 100 is applied in a stripe shape on the application region, a pattern C in which the cooling sensation material 100 is applied so as to broaden toward the outside of the application region (namely in Fig. 18(a), toward the outside in the width direction of the absorbent article, and in Fig. 18(b), toward the outside in the longitudinal direction of the absorbent article), and a pattern D in which the same is applied intermittently in the flow direction.

Specifically in the pattern C, the width of broadest application region is 8 mm, the width of the second broadest application region is 3 mm, the width of the narrowest application region is 1mm, and the width between each mutual application region is 3 mm. In the portion with the greatest amount applied, the applied amount of the cooling sensation material 100 is 0.1 to 1.0 g/m², preferably 0.3 to 0.8 g/m². In the portion with the little amount applied, the applied amount of the cooling sensation material 100 is 0.01 to 0.3 g/m², preferably 0.05 to 0.2 g/m².

In this embodiment, on the outside of the contact region S, there may be a region in which there is a greater amount per unit area of the cooling sensation material 100. Therefore, the cooling sensation material 100 may be arranged in the contact region S.
In such a case, for example, as illustrated in Fig. 20, it is possible to apply in stages using a plurality of coaters 301 and 302 with different amounts that are applied. Application regions PA and PB are formed, as is an application region PC having a smaller amount of the cooling sensation material 100 per unit area than the application regions PA and PB.

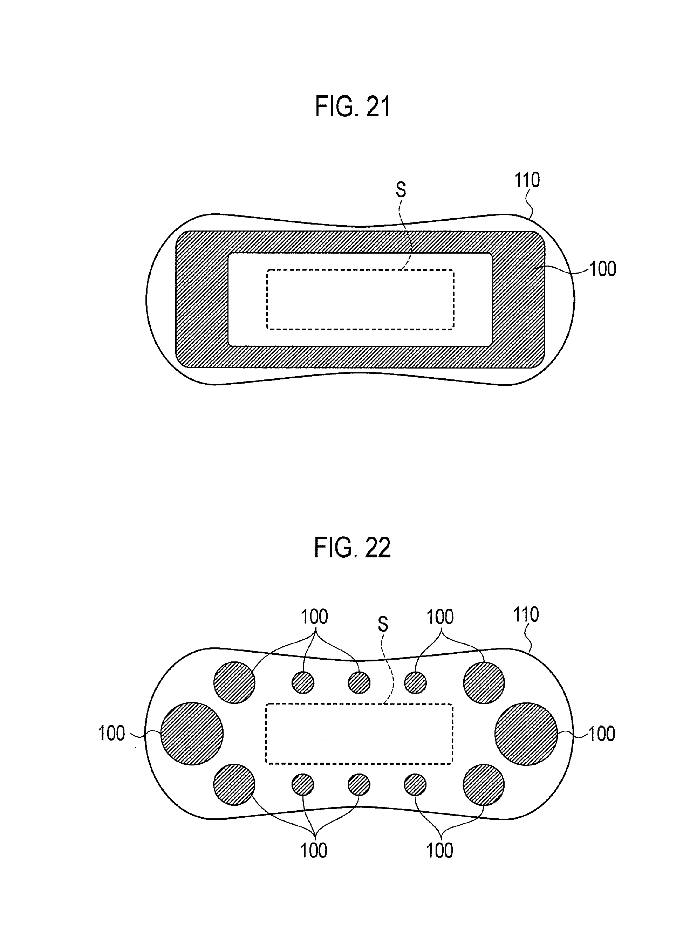

Further, when a so-called flexographic coating method or gravure coating method or the like is used, for example, as illustrated in Fig. 21 and Fig. 22, the cooling sensation material 100 can be arranged at non-continuous positions in the flow direction during the manufacturing of the material 110 configuring the absorbent article.

The cooling sensation material 100 that can be arranged using the second arrangement method and in which the refresher agent is in a solution state is always able to release the refresher agent. Therefore, it is always useful for when there is a desire to exert the refreshing action.

As has been described above, the content of the present invention has been disclosed by means of the first through fifth embodiments, but the statements and drawings, which make up a portion of this disclosure, are not to be understood as limiting the present invention. The various alternative modes of carrying out the present invention, embodiments, and operational techniques will be apparent to the person having ordinary skill in the art based on this disclosure.

For example, the embodiments can be modified as follows. In each of the above-described embodiments, the absorbent article has been described as being a sanitary napkin, but the embodiments could also be applied to so-called liners or incontinence supplies (such as incontinence pads) and the like.

The absorbent article is also not to be limited to the planar shape disclosed in Fig. 1 described above. The shape may also be made to conform to the shape of the wearer's crotch and to the shape of the shorts. The planar shape of the absorbent article can be made to be rectangular, elliptical, gourd-shaped or the like.

Because the absorbent article prevents side leakage of bodily fluids such as menstrual blood, a gather using an elastic material such as a flexible material may be provided on both ends of the widthwise direction of the absorber.

Generally a granular polymer is used as the polymer absorber, such as a sodium acrylate copolymer, which is absorbent or hygroscopic. Also, in addition to the absorber, polymer absorber, and hygroscopic material, a granular deodorant material such as silver, copper, zinc, silica, activated carbon, aluminosilicate compound, and zeolite, may be arranged between the topsheet and the backsheet.

Some electrolytes that can be utilized as the hygroscopic material such as silver, copper, zinc, silica, activated carbon, aluminosilicate compound, and zeolite include those having efficacy in suppressing microbial breeding (an antibacterial effect or bactericidal project). For example, when an electrolyte having efficacy in suppressing microbial breeding is used as the hygroscopic material, then the efficacy in suppressing microbial breeding can be imparted to the absorbent article.

In the above-described embodiments, the cooling sensation material 100 may be a component in which the refresher agent is carried on a porous material capable of adsorbing molecules into a plurality of pores. The porous material is, for example, silica gel, alumina, zeolite, or a nano-porous material.

In the above-described embodiments, there may be a region between the topsheet 10 and the backsheet 20 and at a position corresponding to the region T illustrated in Fig. 1, in which there is a greater amount of the cooling sensation material 100 per unit area; the position in the thickness direction at which the cooling sensation material 100 is arranged is not limited to the above embodiments. In the above-described embodiments, a description has been provided for when a topsheet 10 and a backsheet 20 are provided and the absorber 30 is arranged between the topsheet 10 and the backsheet 20. It has been described that the absorber 30 may be covered by means of a covering material 31. However the absorbent article may also have yet another sheet (for example, a second sheet) arranged between the topsheet 10 and the absorber 30. In such a case, a cooling sensation material 100 can be arranged between the topsheet and the second sheet using the above-described first or second arrangement method.

When there is overlap with not only the above-described second sheet but also a plurality of sheets, then between the topsheet 10 and the backsheet 20 and at the position corresponding to the region T illustrated in Fig. 1, the closer to the edge 30a of the absorber 30, the more preferable that the cooling sensation material 100 be arranged to the side nearer to the topsheet 10 (the upper side). Because in such a case, the closer to the edge unit 30a of the absorber 30, the closer the position at which the cooling sensation material 100 is located is to the side of the wearer's skin, then a stronger refreshing action is imparted. Therefore, the function as an indicator can be more effectively exerted.

In this manner, the present invention, of course, includes various modes and the like that have not be recited herein. Therefore, the technical scope of the present invention is to be defined only by the specific inventive items according to the claims validated from the above description.

The entire contents of Japanese Patent Application No. 2009-088494 (filed on March 31, 2009) are incorporated in the present specification by way of reference.

### [Industrial Applicability]

According to the present invention, it is possible to prevent an excessive stimulation from being given to the wearer by the refresher agent, and also to more reliably prevent the sense of discomfort from steaminess or stickiness.

## Claims

1. An absorbent article, comprising:
a topsheet having a contact surface in contact with the skin of a wearer;
a liquid-impermeable backsheet through which a liquid does not pass; and
an absorber provided between the topsheet and the backsheet, the absorbent article, further comprising:
a cooling sensation material including a refresher agent in the topsheet or between the topsheet and the backsheet, wherein
on the outside in the planar direction of the topsheet from the contact region in which an excretion unit of the wearer is in contact in the topsheet, there is a region having a greater amount of the cooling sensation material per unit area than the amount of the cooling sensation material per unit area in the contact region.

2. The absorbent article according to claim 1, wherein the cooling sensation material is arranged at least on the inside in the widthwise direction from both sides of the absorber in the widthwise direction, and on the outside of an edge unit of the contact region in the widthwise direction.

3. The absorbent article according to claim 2, wherein the amount of the cooling sensation material per unit area from the edge unit of the contact region in the widthwise direction is greater farther toward the edge unit of the absorber in the widthwise direction.

4. The absorbent article according to claim 1, wherein the cooling sensation material is arranged on the inside in the longitudinal direction from the edge unit of the absorber in the longitudinal direction, and on the outside of the edge unit of the contact region in the longitudinal direction.

5. The absorbent article according to claim 4, wherein, from the edge unit of the contact region in the longitudinal direction, there is a greater amount of the cooling sensation material per unit area farther toward the edge unit of the absorber in the longitudinal direction.

6. The absorbent article according to claim 1, wherein the region where the cooling sensation material is arranged is within a range of 30% or less inside the widthwise direction from the edge unit of the absorber relative to the length of the absorber in the widthwise direction.

7. The absorbent article according to claim 1, wherein the cooling sensation material is arranged between the topsheet and the absorber.

8. The absorbent article according to claim 1, wherein the cooling sensation material is arranged on the inside of the absorber.

9. The absorbent article according to claim 1, wherein the cooling sensation material is arranged between the absorber and the backsheet.

10. The absorbent article according to claim 1, wherein
the topsheet includes a first sheet covering at least the top surface of the wearer side of the absorber, and a second sheet arranged on both sides of the first sheet in the widthwise direction,
the second sheet includes a second sheet edge unit located on the inside of the second sheet in the width direction and a folded-back unit located outside of the second sheet edge unit in the widthwise direction of the second sheet,
with the second sheet overlapping onto the first sheet such that the edge unit of the first sheet in the widthwise direction and the edge unit of the second sheet are joined together, a contact surface that is in contact with the skin of the wearer is formed by the folded-back unit being folded-back toward the outside of the first sheet in the widthwise direction, and
the cooling sensation material is arranged between the edge unit of the second sheet and the folded-back unit.
